# EUROPEAN PATENT SPECIFICATION

(11) **EP 1 789 025 B1**
(45) Date of publication and mention of the grant of the patent: **21.04.2010**
(21) Application number: 05771775.3
(22) Date of filing: 22.07.2005
(51) Int. Cl.: A61K 9/70

(54) **DEVICE FOR TRANSDERMAL DELIVERY OF ACTIVE PRINCIPLES**
VORRICHTUNG FÜR DIE TRANSDERMALE ABGABE VON WIRKSTOFFEN
DISPOSITIF POUR L'ADMINISTRATION TRANSDERMIQUE DE PRINCIPES ACTIFS

(30) Priority: 23.07.2004 IT MI20040149
(43) Date of publication of application: 30.05.2007
(73) Proprietor: LABORATORIO ITALIANO BIOCHIMICO FARMACEUTICO LISAPHARMA S.P.A., 22036 Erba (Como) (IT)
(72) Inventor: COLOMBO, Paolo, I-43100 PARMA (IT); SANTI, Patrizia, I-43039 SALSOMAGGIORE (IT); NICOLI, Sara, I-29100 PARMA (IT); PADULA, Cristina, I-85018 TRIVIGNO (IT); MARRA, Fabio, I-73047 MONTERONI DI LECCE (IT)
(74) Representative: Gervasi, Gemma
(86) International application number: PCT/EP2005/053581
(87) International publication number: WO 2006/008320

(56) References cited:
- WO-A-02/30402
- US-A- 5 393 529
- US-A- 5 456 745
- KANIKKANNAN N ET AL: "TRANSDERMAL DELIVERY OF INDOMETHACIN: I. RELEASE PROFILE OF DRUG FROM POLYMERIC PATCHES" INDIAN DRUGS, vol. 30, no. 9, 1993, pages 441-445, XP008002545
- WOOLFSON A D ET AL: "Development and characterisation of a moisture-activated bioadhesive drug delivery system for percutaneous local anaesthesia" INTERNATIONAL JOURNAL OF PHARMACEUTICS, AMSTERDAM, NL, vol. 169, no. 1, 30 June 1998 (1998-06-30), pages 83-94, XP002272600 ISSN: 0378-5173
- ZHAI HONGBO ET AL: "Occlusion vs. skin barrier function." SKIN RESEARCH AND TECHNOLOGY : OFFICIAL JOURNAL OF INTERNATIONAL SOCIETY FOR BIOENGINEERING AND THE SKIN (ISBS) [AND] INTERNATIONAL SOCIETY FOR DIGITAL IMAGING OF SKIN (ISDIS) [AND] INTERNATIONAL SOCIETY FOR SKIN IMAGING (ISSI). FEB 2002, vol. 8, no. 1, February 2002 (2002-02), pages 1-6, XP002370249 ISSN: 0909-752X

## Description

### FIELD OF THE INVENTION

The present invention relates to a device for the delivery of active principles for dermal and, in particular, transdermal application.

### BACKGROUND OF THE INVENTION

The administration of drugs through the skin, known as transdermal administration, has undergone a considerable boost in recent years, by virtue of the development of new systems for delivering a substance to the skin, where the term system means a device formed of various individual elements which jointly contribute to the functioning of the system itself. These delivery systems or devices for the skin are known as dermal or transdermal patches, being composed of an adhesive, supported by a backing which is designed to maintain the drug in contact with the skin.

A typical patch for dermal or transdermal application consists of various elements which are formed in layers of various materials, placed one over the other.

Said layers, superimposed in the following sequence, can be typically:
1. a backing of various materials, which is a transparent or opaque flexible film. It acts as the supporting structure of the patch itself and gives consistency to the patch, allowing it to be positioned and maintained in contact with the skin;
2. a deposit of solid, semisolid or liquid active principle, containing the active substance;
3. a membrane interposed between the active principle deposit and the skin, with the task of controlling active substance release rate. This element is not always present;
4. an adhesive which ensures contact between the patch and the skin surface. Said adhesive must be permeable to the active principle.

In general, all transdermal patches are packaged in foil sachets, the adhesive being covered by a sheet of material (release liner), coated with silicone polymers or fluoropolymers, to avoid unwanted adhesions during patch handling and storage. This element is not involved in the therapeutic application of the patch and is removed before applying to the skin.

From an application viewpoint, transdermal patches are a very practical pharmaceutical form relative to traditional forms of administration. Indeed, the transdermal administration method has many advantages compared to conventional means, such as parenteral and oral means. In the first place, it enables gastro-intestinal metabolism and the hepatic first-pass effect to be avoided; in addition, it is a non-invasive method which allows plasma levels of active principle to be maintained constant for long periods of time, comparable to those following an intravenous infusion. The use of patches for transdermal administration of active principles, however, is considerably limited by the inevitable presence of a latent period prior to the onset of the pharmacological effect. This latent period before the appearance of the therapeutic action can even reach 10 hours. This inconvenient latent period, prior to substance transportation through the skin, is related to patch and skin characteristics. All commercially available patches have this shortcoming.

The current inventors have recently developed a substance delivery device for dermal and transdermal use which is innovative compared to the aforesaid traditional devices. Said device, having been described in patent W002/030402 by the same applicants, consists of a single layer of material in which are included all the elements that constructionally characterise a transdermal patch, that is to say active principle, adhesive and film-forming or structuring agent, that provides the mechanical structure.

The device appears as a resistant film and, being a single layer containing active principle/adhesive/structuring agent, has the same composition on both sides. Moreover the device in question is soluble in water, it is not adhesive as such but becomes adhesive by interacting with water which wets the skin or the device itself.

In recent studies, the current inventors have found that the single layer of WO02/030402 enables to achieve a transport of active principle through the skin which is very rapid and shows much shorter lag times compared to those of traditional patches.

However, said device still presents a series of drawbacks which substantially limit its use for transdermal administration of active principles.

Firstly, in the case of transdermal application, it is desirable that active principle transport be maintained constant for long periods of time i.e. more than 24 hours. Instead, with the device described in WO02/030402 it is observed that, after applying to the skin, the transport of active principle through the skin slows down progressively,and stops after a period of about 24 hours.

Moreover, a further limitation to using said device for transdermal drug administration is the absence of protection on the outer surface i.e. that not in contact with the skin. In this respect, when used with drugs for which transdermal administration is intended, the possibility that one side of the layer could disperse active principle into the environment or onto any clothes with which it comes into contact may not be acceptable. It is therefore necessary to find a means of insulating said exposed surface without modifying the application method of the device, achieved by using water to wet the skin in the area of application.

Therefore, in order to present the transdermal use of said bioadhesive film, a solution to the aforesaid drawbacks must be found.

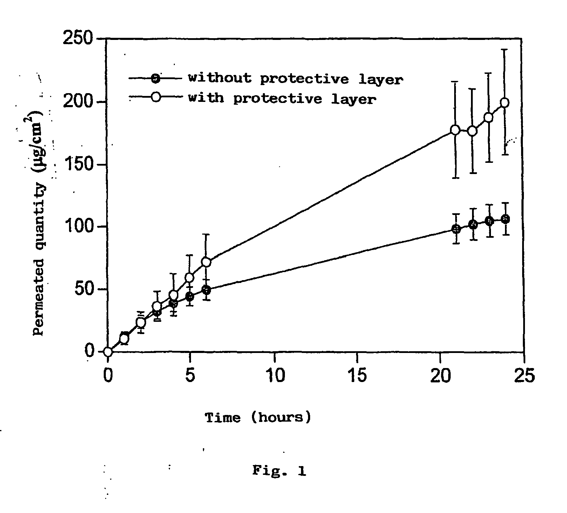

US5456745 discloses gel compositions
0.5 -30% by weight of at least one water soluble polymer being anion active at neutral pH,
0.5-50% by weight of at least one water soluble being cation active at neutral pH,
0.1-70%of water,
0-75% of moisturiser,
0-50% of water soluble or water dispersible auxiliaries,
0-50% of active substance which may be used for preparing the several types of patches disclosed in figures 1-12, and among them, these gel compositions may be used for preparing a bi-layer patch like that disclosed in figure 1 wherein the first layer is formed by the partially dried gel (having in most cases a residual humidity of 25%.

US 5393529 discloses a plaster formed by a first layer containing the acitve ingredient a pressure sensitive adhesive and a water swellable polymer.

### SUMMARY OF THE INVENTION

The current inventors have now discovered that the aforesaid problems encountered with the device described in WO02/030 can be solved by covering one of the two surfaces of said device with a protective layer able to limit water evaporation. The device obtained in this manner, when applied to the skin by the procedures already described, is capable of producing active principle transport kinetics which are completely original and especially favourable when compared to those of both traditional transdermal patches and of the same device with no protection.

### DESCRIPTION OF THE FIGURES

Figure 1 shows the permeation profile of lidocaine from the patch obtained with the procedure described in example 1 a or from a patch in accordance with WO03/020402, having composition identical to that of the active layer of the patch in example 1 a but with no insulating layer.
Figure 2 shows the permeation profile of diclofenac from the patch obtained with the procedure described in example 2a, from a commercially available patch (Flector^{®}, Bayer) and from a patch in accordance with WO03/020402, having composition identical to that of the active layer of the patch in example 2a but with no insulating layer.
Figure 3 shows a permeation profile of estradiol from the patch obtained with the procedure described in example 3a and from a commercially available patch (Estraderm MX^{®}, Novartis Farma).

### DETAILED DESCRIPTION OF THE INVENTION

The present invention concerns a two-layered patch for the dermal or transdermal administration of one or more active principles consisting of a first layer having a homogeneous composition and comprising at least an active principle, a water-soluble film-forming agent and a hydrophilic adhesive polymer, and of a second layer joined in a permanent manner to the first and having a water vapour permeability of less than 500 g/m² in 24 hours.

In particular, in the patch of the present invention, the active principle/adhesive/film-forming polymer mixture, which constitutes the first layer, is spread onto a film of material which has a solely insulating function, and is then dried until the surface exposed to air loses its adhesiveness.

Therefore, the patch of the invention consists of a first layer containing the active principle, known as the active layer, which is not self adhesive, as is the case with normal transdermal patches, but acquires adhesiveness only after contact with water, and of a second layer, known as the insulating layer, which restricts water evaporation from the active layer after the latter is applied to the skin.

The active layer has a thickness preferably between 10 and 1000 µm. The term 'active principle' according to the present invention means any substance with pharmacological or cosmetic activity. However, in accordance with a particularly preferred embodiment of the patch of the present invention, the active principle is preferably a drug.

The film-forming agent and the adhesive polymer can be present in the active layer as two distinct components or, in the case of polymers which possess both properties, as a single component.

Generally, the hydrophilic adhesive is chosen from the group consisting of polyvinylpyrrolidone preferably of molecular weight between 2,000 and 1,500,000, tragacanth gum, gum arabic, karaya gum, xanthan gum, pectin and polyaminomethacrylate adhesives.

According to a preferred embodiment of the present invention the adhesive is selected from the group consisting of polyvinylpyrrolidone, polyaminomethacrylate and mixtures thereof. Of the polyaminomethacrylate adhesives, particularly preferred is the butylmethacrylate/ (2-dimethylaminoethyl)-methacrylate/ methylmethacrylate copolymer in which the ratio between the monomers is 1:2:1, and is commercially known by the name Eudragit E100.

The term 'film-forming agent' according to the present invention means an agent capable of forming a film after being dried.

The water soluble film-forming agent used in the patch of the present invention is generally chosen from the group consisting of carboxymethylcellulose, chitosan, aqueous dispersions of acrylic and methacrylic polymers and polyvinyl alcohol.

According to a more preferred embodiment said film forming agent is polyvinlylacohol and more preferably said polyvinylalcohol is characterized by having a molecular weight between 500 and 115,000 and degree of hydrolysis between 86 and 99%.

The insulating layer consists preferably of a film of polyethylene, polyolefin, ethylene vinyl acetate, polyurethane, polyester, polypropylene, polyvinyl chloride, aluminium, fabric or cross-linked polyvinyl alcohol. Preferably said film has a water permeability less than 250g/m² in 24 hours.

The patch of the invention can also include at least one substance acting as absorption promoter and/or humectant and/or plasticiser. Preferably said substance is chosen from the group consisting of glycerine, ethyl alcohol, propylene glycol, polyethylene glycol of molecular weight from 400 to 6000, sorbitol, phospholipids, terpenes, soya lecithin, phosphatidylcholine, cholesterol, cyclodextrin, isopropyl myristate, oleic acid, polysorbate 80 and diethylene glycol monoethyl ether (commercially known by the name Transcutol^{®}, Gattefossèe, France).

The active layer of the patch of the present invention also contains water in a maximum quantity of 20%, preferably of from 1 to 15%.

Upon use, the non-insulated surface of the patch of the present invention is applied to the skin with water, for example by wetting the skin before applying the patch or by briefly immersing the patch in water, whereas the other surface of the patch remains insulated from the exterior by means of an insulating layer, which limits water evaporation. The patch applied to the skin in this manner remains compact and attaches firmly to the skin. The patch of the present invention precisely covers all the skin surface of application and adapts perfectly to skin wrinkles and folds, considerably increasing surface area of contact and thus transport of active principle into the skin.

As will be illustrated in greater detail by the experimental examples to follow, due to the effect of the composition of the active principle/adhesive/filmogen mixture and the presence of the insulating layer, the patch of the present invention gives rise to kinetics of substance transport through the skin which are particularly favourable when compared with those typical of both normal self-adhesive transdermal patches and of the single layer described in WO02/030402. In particular, unlike that observed with traditional patches, initial transport of a substance through the skin is very rapid and the lag time is practically eliminated. In addition, the duration of active principle transport is considerably greater than that observed with the single layer of WO02/030402 which does not possess an insulating layer. Finally, with the two-layered patch of the present invention, the amount of active principle transported through the skin is considerably greater, for the same duration of application, than that transported in the case of either traditional self-adhesive patches or of the single layer of WO02/030402

The patch of the present invention is useful for the dermal or transdermal administration of any active principle, whether hydrophilic or lipophilic in nature. However, in view of its behaviour in terms of kinetics of active principle transport through the skin, the patch of the present invention is particularly suitable for the transdermal administration of active principles.

Numerous drugs can produce a rapid effect, benefiting from this original transport behaviour, for example the analgesics fentanyl, butorphanol, morphine, buprenorphine, naloxone, codeine; local anaesthetics such as lidocaine, anti-acne drugs like retinoic acid; anti-angina drugs like nitroglycerin, isosorbide dinitrate, nifedipine, nicardipine; antiarrhythmics like timolol; antibacterials like amikacin, cephalosporins, macrolides, tetracyclines, quinolones, nitrofurantoin; anti-convulsives like carbamazepine, phenobarbital, nitrazepam; antidepressants like tricyclics, bupropion, sertraline, pergolide, fluoxetine; anti-rheumatics like diclofenac, ibuprofen, piroxicam, ketoprofen, thiocolchicoside, methotrexate; sex hormone like progesterone, testosterone, estradiol, levonorgestrel; anti-fungals like clotrimazole, ketoconazole, miconazole; anti-hypertensives like sotalol, alprenolol, captopril, enalapril, felodipine, nicardipine, reserpine; anti-hypothyroid drugs like thyroxine; anti-malarials like artemesine, cinchonidine, primaquine; antimigraine drugs like ergotamine, sumatriptan, rizatriptan; anti-nausea drugs like domperidone, chlorpromazine, methoclopramide, scopolamine, tetrahydrocannabinoids; skin lighteners like hydroquinone, hydroquinine; dopamine receptor antagonists like pergolide, bromocriptine; muscle relaxants like thiocolchicoside, diazepam; sclerosing agents like ethanolamine, sodium ricinoleate; vitamins like A, B, C, E and precursors or various agents like oxybutynin, finasteride, erythropoetine.

A further aspect of the present invention is a process for preparing the patch of the present invention comprising the following steps:
a) a mixture is prepared, in water, comprising the hydrophilic adhesive, preferably in an amount between 1% and 50% w/w, the film-forming agent, preferably in an amount between 1 % and 60% w/w, the active principle, preferably in an amount 0.1 % and 20% w/w and, optionally, one or more substances with the properties of absorption promoters/humectants/plasticisers, preferably in an amount between 0.5% and 20%. The water content in said mixture is preferably between 50% and 85%.
b) the mixture obtained in step a) is spread in a thin layer, preferably between 30µm and 3000µm in thickness, onto an insulating film;
c) the patch is dried until a residual moisture content of less than 20% is achieved.

Preferably the preparation of the mixture in step a) is carried out by the following steps:
a1) an aqueous solution of the film-forming agent is prepared;
a2) the hydrophilic adhesive polymer and the active principle are added to the solution obtained in step a1 in the form of an aqueous solution, micronized particles or emulsion.

### COMPARATIVE EXAMPLE 1

### 1a) Preparation of a lidocaine hydrochloride-containing patch non-adhesive in the dry state.

A mixture having the following composition is prepared:

| | |
|---|---|
| lidocaine hydrochloride | 2.00 g |
| PVA 83400, degree of hydrolysis 87.5% | 12.4 g |
| lauric acid | 2.48 g |
| adipic acid | 0.49 g |
| Eudragit E100 | 4.29 g |
| glycerine | 0.27 g |
| sorbitol | 2.8 g |
| water | remainder to 100 g |

In detail, the PVA is hydrated in 49 ml water for 12 hours. It is then gradually heated to 90°C and stirred until completely dissolved. Separately, the adhesive is prepared by adding Eudragit E100, lauric acid and adipic acid to 21.27 ml of water, previously heated to a temperature of 78-82°C. The mixture is stirred for about 30 minutes, maintaining the temperature constant. The mixture is subsequently cooled to 60°C and 0.27 g of glycerine are added. In another container the lidocaine hydrochloride is dissolved in 5 ml of water. The adhesive solution, the lidocaine solution and the sorbitol are added to the PVA solution in that order.

The mass obtained is spread as a thin film (300 µm thickness) using a doctor blade device, such as that supplied by BYK - Gardner (Silver Spring, USA), onto an occlusive protective layer (Scotchpak 1220, 3M, USA). The entirety is placed in a ventilated oven for 30 minutes at a temperature of 80°C. The patch obtained is one layer of 5.0 µm thickness containing a quantity of lidocaine equal to 0.4 mg/cm².

### 1b) In vitro permeation experiments through rabbit ear skin

The in vitro permeation of a drug from patches prepared in example 1a) was analysed using a vertical Franz diffusion cell and a barrier consisting of rabbit ear skin, in accordance with the protocol described by Cristina Padula et al, in Journal Controlled Rel 88, 277-285, 2003.

Patches without an insulating layer and consisting only of the active layer of the patches prepared in example 1a), prepared as described in WO02/030402, were used as controls.

Figure 1 shows the average quantity of lidocaine permeated with time per cm² of patch for each of the two types of patches tested.

The data obtained indicate that both patches tested give rise to a very rapid active principle transport through the skin, with no lag time.

However, in the case of the patch of the present invention, that is to say in the presence of the protective layer, the total amount of lidocaine permeated as well as the duration of drug transport through the skin are considerably greater than with the single layer patch.

### COMPARATIVE EXAMPLE 2

### 2a) Preparation of a diclofenac potassium-containing patch non-adhesive in the dry state

A mixture is prepared having the following composition:

| | |
|---|---|
| diclofenac potassium | 4.72 g |
| PVA 83400, degree of hydrolysis 87.5% | 1.13 g |
| PVP K 90 | 14.47 g |
| PEG 400 | 8.14 g |
| Lutrol F 127 | 3.85 g |
| Eugenol | 3.39 g |
| Menthol | 2.71 g |
| water | remainder to 100 g |

In detail, the PVA is hydrated in 4.52 ml of water for 12 hours. It is then gradually heated to 90°C and stirred until completely dissolved. Separately, a solution of Lutrol F127 (Basf, Germany) is prepared, dispersing the triblock copolymer in 12.22 ml of water, leaving under agitation at ambient temperature for about one hour then maintaining the solution obtained at about 4°C for at least 2 hours. 1.29 g of the drug are added at ambient temperature while stirring. Separately, the adhesive is prepared by slowly adding the PVP K 90 (Basf, Germany) to a solution of PEG 400 in 44.85 ml of water, and stirring gently for 12 hours to favour polymer hydration. The components are mixed together, the remaining quantity of drug is added and, once dissolved, the eugenol and the menthol are added to the mass. The mixture is slowly stirred for one hour at 60°C, to favour mixing of the components. The mass obtained is spread in the form of a thin film (600 µm thickness) using a doctor blade device, such as that supplied by BYK Gardner (Silver Spring, USA) onto an occlusive backing sheet (Cotran 9702, 3M, USA). The patch obtained is one layer of 40 µm thickness containing a quantity of diclofenac equal to 2.5 mg/cm².

### 2b) In vitro permeation experiments through rabbit ear skin

The in vitro permeation of a drug from patches prepared in example 2a) was analysed using a vertical Franz diffusion cell and a barrier consisting of rabbit ear skin (Cristina Padula et al, in Journal Controlled Rel 88, 277-285, 2003). Commercially available patches containing 1 mg/cm² diclofenac (Flector ^{®}, Bayer) and patches consisting of only the active layer of the patches prepared in example 2, prepared as described in WO 02/030402, and without insulating layer, were used as controls.

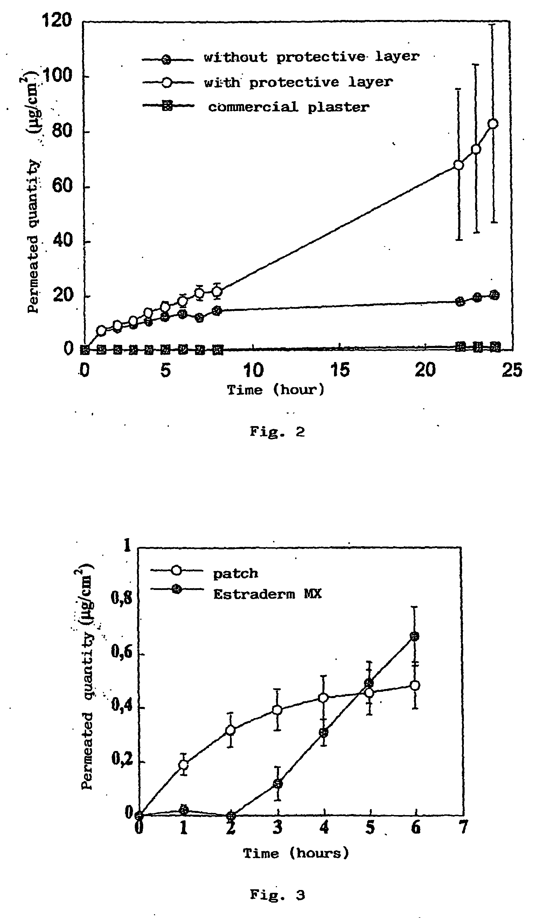

Figure 2 shows the average quantity of diclofenac permeated with time per cm² of patch for each of the three types of patches tested.

The results obtained demonstrate that the patch of the present invention has favourable characteristics compared to both commercially available patches containing the same active principle and the patches described in WO02/030402.

In fact, compared to commercially available patches where a lag time of some hours and very low amounts of permeated drug are observed, the patches of the present invention provide a very rapid initial transport through the skin with no lag time and greater amounts of transported drug, this being a characteristic also observed with the patch of WO02/030402.

Furthermore, compared to the patches of WO02/030402, the patches of the present invention have the further advantage of providing a transport duration that is considerably extended as well as a greater quantity of transported drug. In particular, while, in the case of the WO02/030402 patches drug transport slows down gradually over time and stops after 8 hours, with the patches of the present invention such transport is sustained for over 24 hours.

### COMPARATIVE EXAMPLE 3

### a) Preparation of an estradiol-containing patch non-adhesive in the dry state

A mixture is prepared having the following composition:

| | |
|---|---|
| estradiol | 0.08 g |
| PVA 83400, degree of hydrolysis 87.5% | 12.40 g |
| lauric acid | 2.48 g |
| adipic acid | 0.49 g |
| Eudragit E 100 | 4.29 g |
| glycerine | 7.17 g |
| β-cyclodextrin | 0.40 g |
| water | remainder to 100 g |

In detail, the PVA is hydrated for 12 hours in 49 ml of water. It is then gradually heated to 90°C and stirred until completely dissolved. Separately, the adhesive is prepared by adding Eudragit E 100, lauric acid and adipic acid to 19.47 ml of water, previously heated to a temperature of 78-82°C. The mixture is stirred for about 30 minutes, maintaining the temperature constant. The mixture is subsequently cooled to 60°C and 7.17 g of glycerine are added. In another container the estradiol and cyclodextrin are dissolved in 4.22 ml of water in another container. The adhesive solution and the estradiol solution are added to the PVA solution in that order.

The mass obtained is spread in the form of a thin film (400 µm thickness) using a doctor blade, such as that supplied by BYK - Gardner (Silver Spring, USA), onto an occlusive backing sheet (Scotchpak 1220, 3M, USA).

The patch obtained is one layer of 60 µm thickness containing a quantity of lidocaine equal to 7.5 µg/cm².

### b) In vitro permeation experiments through rabbit ear skin

The in vitro permeation of a drug from patches prepared in example 3a) was analysed using a vertical Franz diffusion cell and a barrier consisting of rabbit ear skin (Cristina Padula et al, in Journal Controlled Rel 88, 277-285, 2003).

Commercially available patches each containing 68.2 µg/cm² of estradiol (Estraderm MX^{®}) were used as a control.

Figure 3 shows the average quantity of estradiol permeated with time per cm² of patch for each of the two types of patch tested. The results obtained demonstrate that the patch of the present invention, contrary to commercial patch, provides an immediate drug release with no lag time.

## Claims

1. Two-layered patch for dermal or transdermal administration of active principles consisting of:
a) a first layer having a homogeneous composition and consisting of
- at least one active principle,
- a water-soluble film-forming agent selected from the group consisting of carboxymethylcellulose, chitosan, aqueous dispersions of acrylic and methacrylic polymers and polyvinyl alcohol,
- a hydrophilic adhesive polymer, chosen from the group consisting of polyvinylpyrrolidone, tragacanth gum, arabic gum, karaya gum, xanthan gum, pectin and, polyaminomethacrylate adhesives,
- at least one substance acting as absorption promoter and/or humectant and/or plasticiser chosen from the group consisting of glycerine, ethyl alcohol, propylene glycol, polyethylene glycol of molecular weight between 400 and 6000, sorbitol, phospholipids, terpenes, soya lecithin, phosphatidylcholine, cholesterol, cyclodextrin, isopropyl myristate, oleic acid, polysorbate 80 and diethylene glycol monoethyl ether,
said first layer (a) having a residual water content lower than 20%;
b) a second layer joined in a permanent manner to the first and having a permeability to water vapour of less than 500 g/m² in 24 hours,
provided that:
when said adhesive in said layer (a) is polyaminomethacrylate and it is butylmethacrylate/ (2-dimethylaminoethyl)-methacrylate/ methylmethacrylate copolymer in which the ratio between the monomers is 1:2:1, said water-soluble film-forming agent must be different from carboxymethylcellulose.

2. Patch as claimed in claim 1 wherein said residual water content is comprised between 1 and 15%.

3. Patch as claimed in anyone of claims 1-2, wherein said adhesive is selected from the group consisting of polyvinylpyrrolidone, polyaminomethacrylate and mixtures thereof.

4. Patch as claimed in anyone of claims 1-3, wherein the average molecular weight of polyvinyl pyrrolidone is comprised between 2,000 and 1,500,000.

5. Patch as claimed in anyone of claims 1-3 wherein said polyaminomethacrylate is butylmethacrylate/ (2-dimethylaminoethyl)-methacrylate/ methylmethacrylate copolymer in which the ratio between the monomers is 1:2:1.

6. Patch as claimed in anyone of claims 1-5, wherein said film forming agent is polyvinylalcohol.

7. Patch as claimed in any one of claims 1-6, wherein said polyvinylalcohol has a molecular weight between 500 and 115,000 and degree of hydrolysis between 86 and 99%.

8. Patch as claimed in anyone of claims 1-7 wherein said second layer (b) consists of polyethylene, polyolefin, ethylene vinyl acetate, polyurethane, polyester, polypropylene, polyvinyl chloride, cross-linked polyvinyl alcohol, aluminium or fabrics.

9. Patch as claimed in anyone of claims from 1 to 8, wherein the first layer has a thickness of between 10 and 1000µm.

10. Process for preparing a patch in accordance with anyone of claims 1-9, comprising the following steps:
a) a mixture is prepared, in water, comprising the hydrophilic adhesive, the film-forming agent, the active principle, and one or more substances with the properties of absorption promoters/humectants/plasticisers;
b) the mixture obtained in step a) is spread in a thin layer onto an insulating film;
c) the patch is dried until a residual water content of less than 20% is achieved.

11. Process as claimed in claim 10, wherein the mixture prepared in step a) contains between 1% and 50% w/w of hydrophilic adhesive, between 1% and 60% w/w of film-forming agent, between 0.1 % and 20% w/w of active principle, between 50% and 85% w/w of water, and between 0.5% and 20% w/w of one or more substances with the properties of absorption promoters/humectants/plasticisers.

12. Process as claimed in claim 10 or 11, wherein the mixture of step a) is prepared by a process comprising the following steps:
a1) an aqueous solution of the film-forming agent is prepared;
a2) a solution of hydrophilic adhesive polymer and the active principle in the form of an aqueous solution, micronized particles or emulsion is added to the solution obtained in step a1).

13. Process as claimed in anyone of claims 10 to 12, wherein in step b) said mixture is spread to give a layer of thickness between 30 and 3000µm.

## Patentansprüche

1. Zweischichtiges Pflaster für die dermale oder transdermale Verabreichung von Wirkstoffen, bestehend aus:
a) einer ersten Schicht mit einer homogenen Zusammensetzung und bestehend aus
- mindestens einem Wirkstoff;
- einem wasserlöslichen folienbildenden Bestandteil, ausgewählt aus der Gruppe bestehend aus Carboxymethylcellulose, Chitosan, wässrigen Dispersionen von Acryl- und Methacrylpolymeren und Polyvinylalkohol;
- einem hydrophilen Polymer-Haftmittel, ausgewählt aus der Gruppe bestehend aus Polyvinylpyrrolidon-, Tragacanthgummi-, Gummiarabikum-, Karayagummi-, Xanthangummi-, Pektin- und Polyaminomethacrylat-Haftmitteln;
- mindestens einer Substanz, welche als Absorptionpromoter und/oder Feuchthaltemittel und/oder Weichmacher wirkt, ausgewählt aus der Gruppe bestehend aus Glycerin, Ethylalkohol, Propylenglycol, Polyethylenglycol mit einem Molekulargewicht zwischen 400 und 6000, Sorbitol, Phospholipiden, Terpenen, Sojalecithin, Phosphatidylcholin, Cholesterin, Cyclodextrin, Isopropylmyristat, Oleinsäure, Polysorbat 80 und Diethylenglycolmonoethylether, wobei die genannte erste Schicht (a) einen Restwassergehalt von weniger als 20 % aufweist;
b) einer zweiten Schicht, welche auf dauerhafte Art und Weise mit der ersten Schicht verbunden ist und welche eine Permeabilität für Wasserdampf von weniger als 500g/m² in 24 Stunden aufweist, unter der Voraussetzung, dass:
wenn das genannte Haftmittel in genannter Schicht (a) ein Polyaminomethacrylat ist und es sich dabei um Butylmethacrylat/ (2-dimethylaminoethyl)-methacrylat/ methylmethacrylat-Copolymer handelt, in welchem das Verhältnis zwischen den Monomeren 1:2:1 beträgt, der genannte wasserlösliche folienbildende Bestandteil eine andere Substanz als Carboxymethylcellulose sein muss.

2. Pflaster, wie in Anspruch 1 beansprucht, in welchem der genannte Restwassergehalt zwischen 1 und 15 % liegt.

3. Pflaster, wie in einem der Ansprüche 1-2 beansprucht, in welchem das genannte Haftmittel aus der Gruppe ausgewählt ist, bestehend aus Polyvinylpyrrolidon, Polyaminomethacrylat und Mischungen daraus

4. Pflaster, wie in einem der Ansprüche 1-3 beansprucht, in welchem das durchschnittliche Molekulargewicht von Polyvinylpyrrolidon zwischen 2.000 und 1.500.000 liegt.

5. Pflaster, wie in einem der Ansprüche 1-3 beansprucht, in welchem genanntes Polyaminomethacrylat Butylmethacrylat/ (2-dimethylaminoethyl)-methacrylat/ methylmethacrylat-Copolymer ist, in welchem das Verhältnis zwischen den Monomeren 1:2:1 beträgt.

6. Pflaster, wie in einem der Ansprüche 1-5 beansprucht, in welchem der genannte folienbildende Bestandteil ein Polyvinylalkohol ist.

7. Pflaster, wie in einem der Ansprüche 1-6 beansprucht, in welchem genannter Polyvinylalkohol ein Molekulargewicht zwischen 500 und 115.000 besitzt sowie einen Hydrolysegrad zwischen 86 und 99 % aufweist.

8. Pflaster, wie in einem der Ansprüche 1-7 beansprucht, in welchem genannte zweite Schicht (b) aus Polyethylen, Polyolefin, Ethylenvinylacetat, Polyurethan. Polyester, Polypropylen, Polyvinylchlorid, quervernetztem Polyvinylalkohol, Aluminium oder Geweben besteht.

9. Pflaster, wie in einem der Ansprüche 1-8 beansprucht, in welchem die erste Schicht eine Dicke zwischen 10 und 1000 µm aufweist.

10. Verfahren zur Herstellung eines Pflasters gemäß einem der Ansprüche 1-9, welches die folgenden Schritte umfasst:
a) es wird eine Mischung in Wasser hergestellt, umfassend das hydrophile Haftmittel, den folienbildenden Bestandteil, den Wirkstoff und eine Substanz oder mehrere Substanzen mit den Eigenschaften von Absorptionspromotern/Feuchthaltemitteln/Weichmachern;
b) die in Schritt a) erhaltene Mischung wird in einer dünnen Schicht auf eine Isolationsfolie verteilt;
c) das Pflaster wird getrocknet bis der Restwassergehalt weniger als 20 % beträgt.

11. Verfahren, wie in Anspruch 10 beansprucht, wobei die in Schritt a) hergestellte Mischung zwischen 1 % und 50 % w/w an hydrophilem Haftmittel enthält, zwischen 1 % und 60 % w/w des folienbildenden Bestandteils, zwischen 0,1 % und 20 % w/w des Wirkstoffes, zwischen 50 % und 85 % w/w Wasser und zwischen 0,5 % und 20 % w/w von einer Substanz oder mehreren Substanzen mit den Eigenschaften von Absorptionspromotern/Feuchthaltemitteln/Weichmachern.

12. Verfahren, wie in Anspruch 10 oder 11 beansprucht, wobei die Mischung aus Schritt a) mit einem Verfahren hergestellt wird, welches die folgenden Schritte umfasst:
a1) es wird eine wässrige Lösung des folienbildenden Bestandteils hergestellt;
a2) es wird eine Lösung des hydrophilen Polymer-Haftmittels und des Wirkstoffs in der Form einer wässrigen Lösung, in der Form von mikronisierten Partikeln oder als Emulsion zu der in Schritt a1 erhaltenen Lösung hinzugegeben.

13. Verfahren, wie in einem der Ansprüche 10 bis 12 beansprucht, wobei in Schritt b) genannte Mischung verteilt wird, um eine Schichtdicke zwischen 30 und 3000 µm zu ergeben.

## Revendications

1. Timbre à deux couches destiné à une administration dermique ou transdermique de principes actifs, constitué par :
a) une première couche ayant une composition homogène constituée par
- au moins un principe actif,
- un agent filmogène soluble dans l'eau, choisi dans le groupe constitué par la carboxyméthylcellulose, le chitosane, des dispersions aqueuses de polymères acryliques et méthacryliques et d'alcool polyvinylique,
- un polymère hydrophile adhésif, choisi dans le groupe constitué par les adhésifs à base de polyvinylpyrrolidone, de gomme de tragacanthe, de gomme arabique, de gomme de karaya, de gomme de xanthane, de pectine et de polyaminométhacrylate,
- au moins une substance agissant comme un promoteur d'absorption et/ou un humectant et/ou un plastifiant choisi dans le groupe constitué par la glycérine, l'alcool éthylique, le propylène glycol, le polyéthylène glycol d'une masse moléculaire comprise entre 400 et 6000, le sorbitol, les phospholipides, les terpènes, la lécithine de soja, la phosphatidylcholine, le cholestérol, la cyclodextrine, le myristate d'isopropyle, l'acide oléique, le polysorbate 80 et l'éther monométhylique du diéthylène glycol,
ladite première couche (a) ayant une teneur en eau résiduelle inférieure à 20 % ;
b) une deuxième couche jointe de manière permanente à la première et ayant une perméabilité à la vapeur d'eau inférieure à 500 g/m² en 24 heures,
à condition qui :
lorsque ledit adhésif dans ladite couche (a) est un polyaminométhacrylate et qu'il est un copolymère de butylméthacrylate/(2-diméthylaminoéthyl)-méthacrylate/méthylméthacrylate dans lequel le rapport entre les monomères est de 1/2/1, ledit agent filmogène soluble dans l'eau soit différent de la carboxyméthylcellulose.

2. Timbre selon la revendication 1, dans lequel ladite teneur en eau résiduelle est comprise entre 1 et 15 %.

3. Timbre selon l'une quelconque des revendications 1 à 2, dans lequel ledit adhésif est choisi dans le groupe constitué par la polyvinylpyrrolidone, le polyaminométhacrylate et leurs mélanges.

4. Timbre selon l'une quelconque des revendications 1 à 3, dans lequel la masse moléculaire moyenne de la polyvinylpyrrolidone est comprise entre 2 000 et 1 500 000.

5. Timbre selon l'une quelconque des revendications 1 à 3, dans lequel ledit polyaminométhacrylate est un copolymère de butylméthacrylate/(2-diméthylaminoéthyl)-méthacrylate/méthylméthacrylate dans lequel le rapport entre les monomères est de 1/2/1.

6. Timbre selon l'une quelconque des revendications 1 à 5, dans lequel ledit agent filmogène est l'alcool polyvinylique.

7. Timbre selon l'une quelconque des revendications 1 à 6, dans lequel ledit alcool polyvinylique a une masse moléculaire comprise entre 500 et 115 000 et un degré d'hydrolyse compris entre 86 et 99 %.

8. Timbre selon l'une quelconque des revendications 1 à 7, dans lequel ladite deuxième couche (b) est constituée par du polyéthylène, une polyoléfine, du vinyl acétate d'éthylène, du polyuréthane, du polyester, du polypropylène, du chlorure de polyvinyle, de l'alcool polyvinylique réticulé, de l'aluminium ou du tissu.

9. Timbre selon l'une quelconque des revendications 1 à 8, dans lequel la première couche a une épaisseur comprise entre 10 et 1000 µm.

10. Procédé de préparation d'un timbre selon l'une quelconque des revendications 1 à 9, qui comprend les étapes suivantes :
a) la préparation d'un mélange, dans l'eau, qui comprend l'adhésif hydrophile, l'agent filmogène, le principe actif, et une ou plusieurs substances ayant les propriétés de promoteurs d'absorption/d'humectants/de plastifiants ;
b) la formation d'une fine couche du mélange obtenu dans l'étape a) sur un film isolant ;
c) le séchage du timbre jusqu'à l'obtention d'une teneur en eau résiduelle inférieure à 20 %.

11. Procédé selon la revendication 10, dans lequel le mélange préparé dans l'étape a) contient de 1 % à 50 % p/p d'adhésif hydrophile, de 1 % à 60 % p/p d'agent filmogène, de 0,1 % à 20 % p/p de principe actif, de 50 % à 85 % p/p d'eau, et de 0,5 % à 20 % p/p d'une ou plusieurs substances ayant les propriétés de promoteurs d'absorption/d'humectants/de plastifiants.

12. Procédé selon la revendication 10 ou 11, dans lequel le mélange de l'étape a) est préparé par un procédé qui comprend les étapes suivantes .
a1) la préparation d'une solution aqueuse de l'agent filmogène ;
a2) l'ajout à la solution obtenue dans l'étape a1) d'une solution de polymère adhésif hydrophile et du principe actif sous la forme d'une solution aqueuse, de particules micronisées ou d'une émulsion.

13. Procédé selon l'une quelconque des revendications 10 à 12, dans lequel dans l'étape b) ledit mélange est étalé pour obtenir une couche d'une épaisseur comprise entre 30 et 3000 µm.
